# EUROPÄISCHE PATENTSCHRIFT

(11) **EP 3 168 671 B1**
(45) Veröffentlichungstag und Bekanntmachung des Hinweises auf die Patenterteilung: **04.10.2023**
(21) Anmeldenummer: 16195992.9
(22) Anmeldetag: 27.10.2016
(51) Int. Cl.: G02B 21/08

(54) **BELEUCHTUNGSANORDNUNG**
ILLUMINATION ARRANGEMENT
ARRANGEMENT D'ILLUMINATION

(30) Priorität: 12.11.2015 DE 102015119590
(43) Veröffentlichungstag der Anmeldung: 17.05.2017
(73) Patentinhaber: Jung, Carsten, 35614 Aßlar (DE)
(72) Erfinder: Jung, Carsten, 35614 Aßlar (DE)
(74) Vertreter: Hoefer & Partner Patentanwälte mbB

(56) Entgegenhaltungen:
- EP-A1- 2 037 308
- JP-A- 2011 242 609
- US-A- 2 471 879

## Beschreibung

Die Erfindung betrifft ein Diagnose- oder Operationsmikroskop der im Oberbegriff des Anspruchs 1 genannten Art.

Insbesondere bei Diagnose- oder Operationsmikroskopen besteht die Anforderung, ein mittels des Mikroskops zu untersuchendes Objekt ausreichend zu beleuchten. Hierfür wird eine Beleuchtungsanordnung verwendet, deren Beleuchtungsstrahlengang beispielsweise über ein Umlenkelement in den durch das Objektiv des Mikroskops definierten Beobachtungsstrahlengang eingeblendet wird. Gleichzeitig besteht die Anforderung, eine homogene Ausleuchtung zu erzielen.

Beleuchtungsanordnungen für Mikroskope sind beispielsweise durch DE 10 2007 027 084 A1, DE 10 2011 086 178 A1, DE 10 2006 017799 A1 und DE 101 44 062 A1 bekannt.

Durch DE 103 47 732 A1 ist eine Beleuchtungsanordnung für ein Mikroskop bekannt, die eine Lichtquelle und eine zwischen der Lichtquelle und einem Objektiv des Mikroskops angeordnete oder anordenbare Beleuchtungsoptik aufweist.

Durch JP 2011/242609 A ist ein Mikroskop mit einer Beleuchtungsanordnung bekannt, wobei im Abbildungsstrahlengang des Mikroskops ein afokales pankratisches System angeordnet ist.

Ein ähnliches Mikroskop ist auch durch US 2007/0091939 A1 bekannt.

Durch EP 1 153 569 A2 ist ein Operationsmikroskop bekannt, das einen Beleuchtungsstrahlengang aufweist, bei dem durch Entfernen oder Lageveränderung eines optischen Bauteiles am Objekt eine Abdunkelung der Beleuchtung erzielt wird.

Durch DE 103 55 523 A1 ist ein Stereomikroskop bekannt, bei dem sowohl im Abbildungsstrahlengang als auch im Beleuchtungsstrahlengang ein Zoomsystem angeordnet ist, um eine an den Zoomfaktor des Beleuchtungsstrahlenganges angepasste Beleuchtung zu realisieren.

Durch EP 2 037 308 A1 ist ein Diagnose- oder Operationsmikroskop mit den Merkmalen des Oberbegriffs des Anspruchs 1 bekannt.

Eine ähnliche Beleuchtungsanordnung ist auch durch US 2002/0159143 A1 bekannt.

Der Erfindung liegt die Aufgabe zugrunde, ein Diagnose- oder Operationsmikroskop der im Oberbegriff des Anspruchs 1 genannten Art anzugeben, das eine verbesserte Beleuchtung aufweist.

Diese Aufgabe wird durch die im Anspruch 1 angegebene Erfindung gelöst.

Der Erfindung liegt der Gedanke zugrunde, die Beleuchtungsanordnung so auszugestalten, dass bei Bedarf auf einfache Weise eine Erhöhung der Beleuchtungsstärke bei gleichzeitiger Beibehaltung der Homogenität der Beleuchtung entlang der beleuchteten Fläche im Beobachtungsfeld ermöglicht ist.

Ausgehend von diesem Grundgedanken verwendet die Erfindung eine Köhlersche Beleuchtung, bei der die Lichtquelle über eine Kollektorlinsenanordnung in die Ebene einer Feldblende (Aperturblende) abgebildet wird, wobei der Feldblende eine Hilslinsenanordnung nachgeordnet ist.

Die Hilfslinsenanordnung der Köhler'schen Beleuchtung weist erfindungsgemäß ein pankratisches System (Vario-Objektiv) auf. Das pankratische System ermöglicht eine Veränderung der Brennweite der Beleuchtungsoptik. Wird bei der kürzesten Brennweite des pankratischen Systems der Beleuchtungsoptik eine bestimmte Fläche im Beobachtungsfeld homogen ausgeleuchtet, so verringert sich bei Variation der Brennweite die ausgeleuchtete Fläche, wobei gleichzeitig die Beleuchtungsstärke ansteigt. Verlängert sich beispielsweise bei der Brennweitenverstellung die Brennweite des pankratischen Systems um den Faktor 2, so verkleinert sich der ausgeleuchtete Felddurchmesser linear um denselben Faktor, wobei die geometrische Beleuchtungsstärke um den Faktor 4 ansteigt.

Die Erfindung ermöglicht es damit, bei Bedarf die Beleuchtungsstärke der Beleuchtungsanordnung im Beobachtungsfeld wesentlich zu erhöhen.

Die Erfindung ermöglicht es insbesondere, auch Leuchtdioden mit relativ geringer Beleuchtungsstärke als Lichtquelle einer Beleuchtungsanordnung in einem Diagnose- oder Operationsmikroskop einzusetzen. Damit macht die Erfindung die Vorteile von Leuchtdioden für den Einsatz in Beleuchtungsanordnungen für Mikroskope vollständig nutzbar.

Der Vario-Faktor des pankratischen Systems ist entsprechend den jeweiligen Anforderungen innerhalb weiter Grenzen wählbar.

Gleichermaßen ist das Optikdesign des pankratischen Systems entsprechend den jeweiligen Anforderungen innerhalb weiter Grenzen wählbar.

Vorteilhafte und zweckmäßige Weiterbildungen der Erfindung gemäß Anspruch 1 sind in den abhängigen Ansprüchen angegeben.

Unter einer Optik im Sinne der Erfindung wird eine Anordnung verstanden, die wenigstens ein optisch wirksames Bauelement, insbesondere wenigstens eine optische Linse, aufweist.

Die Erfindung wird nachfolgend anhand eines Ausführungsbeispiels unter Bezugnahme auf die beigefügte stark schematisierte Zeichnung näher erläutert.

Es zeigt:
Fig. 1 stark schematisiert ein Ausführungsbeispiel eines mit einem Ausführungsbeispiel einer erfindungsgemäßen Beleuchtungsanordnung versehenen erfindungsgemäßen Mikroskops,
Fig. 2 stark schematisiert die bei dem Mikroskop gemäß Fig. 1 verwendete Beleuchtungsanordnung bei einer ersten Brennweite des pankratischen Systems und
Fig. 3 in gleicher Darstellung die Fig. 2 die Beleuchtungsanordnung bei einer zweiten Brennweite des pankratischen Systems.

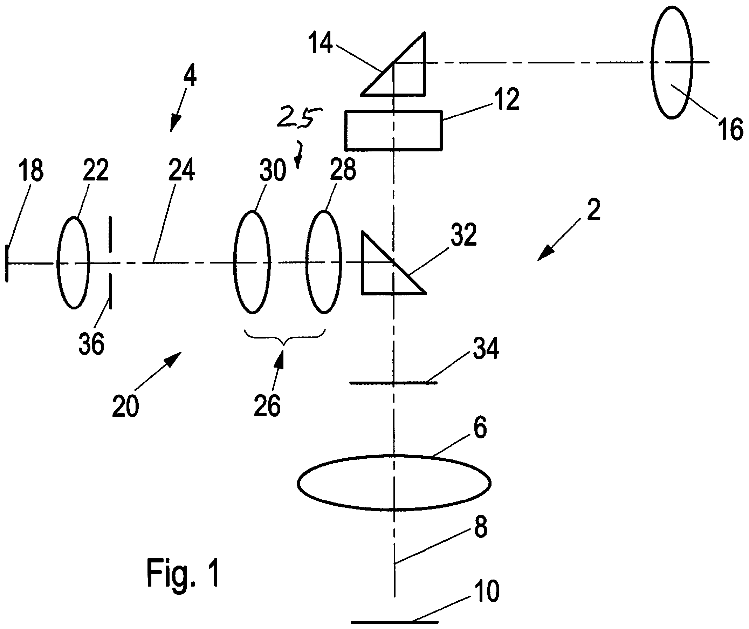

In Fig. 1 ist stark schematisiert ein Ausführungsbeispiel eines erfindungsgemäßen Mikroskops 2 dargestellt, das mit einem Ausführungsbeispiel einer erfindungsgemäßen Beleuchtungsanordnung 4 versehen ist. Die Beleuchtungsanordnung 4 kann je nach Bauform des Mikroskops 2 baulich in dasselbe integriert oder als separate, mit dem Mikroskop 2 verbindbare Baugruppe ausgebildet sein.

Bei dem Mikroskop 2 handelt es sich gemäß der Erfindung um ein Diagnose- oder Operationsmikroskop.

Das Mikroskop 2 weist ein Objektiv 6 (Frontobjektiv) auf, dessen optische Achse einen in Fig. 1 durch eine strichpunktierte Linie symbolisierten Beobachtungsstrahlengang 8 des Mikroskops 2 definiert. Von einem zu beobachtenden Objekt 10 führt der Beobachtungsstrahlengang 8 über einen Vergrößerungswechsler 12 und ein Umlenkprisma 14 zu einem Okular 16 des Mikroskops 2.

Die erfindungsgemäße Beleuchtungsanordnung 4 weist bei dem dargestellten Ausführungsbeispiel eine Lichtquelle 18 in Form einer Leuchtdiode auf.

Die Beleuchtungsanordnung 4 weist ferner eine Beleuchtungsoptik 20 mit einer der Lichtquelle 18 in Strahlrichtung nachgeordneten Kollektor-Linsenanordnung 22 auf, deren optische Achse einen in Fig. 1 ebenfalls durch eine strichpunktierte Linie symbolisierten Beleuchtungsstrahlengang 24 definiert. Der Kollektor-Linsenanordnung 22 ist in Strahlrichtung des Beleuchtungslichts eine Feldblende 36 nachgeordnet, der in Strahlrichtung des Beleuchtungslichts eine vorzugsweise als Kondensor wirkende Hilfslinsenanordnung 25 nachgeordnet ist

Erfindungsgemäß weist die Hilfslinsenanordnung 25 der Beleuchtungsoptik 20 ein pankratisches System 26 (Vario-Objektiv) mit einem Variator 28 und einem Kompensator 30 auf, wobei das pankratische System 26 in Strahlrichtung der Lichtquelle 18 der Kollektor-Linsenanordnung 22 nachgeordnet ist.

Zur Einblendung des Beleuchtungsstrahlenganges 24 in den Beobachtungsstrahlengang 8 des Mikroskops 2 ist ein Umlenkmittel 32 vorgesehen, das dem pankratischen System 26 nachgeordnet und bei diesem Ausführungsbeispiel durch ein Prisma gebildet ist. Das Umlenkmittel 32 kann jedoch auch durch einen Spiegel gebildet sein.

Der grundsätzliche Aufbau und die grundsätzliche Funktionsweise eines pankratischen Systems sind dem Fachmann allgemein bekannt und werden daher hier nicht näher erläutert.

Wie aus Fig. 1 ersichtlich ist, lenkt das Umlenkmittel 32 den Beleuchtungsstrahlengang 24 um 45° um, so dass der Beleuchtungsstrahlengang 24 in den Beobachtungsstrahlengang 8 eingeblendet wird.

In Strahlrichtung zwischen dem Umlenkmittel 32 und dem Objektiv 6 des Mikroskops 2 ist eine optische Vorrichtung 34 zur Verringerung von Reflexionen des Beleuchtungslichts an dem Objektiv 6 angeordnet. Bei dem dargestellten Ausführungsbeispiel ist diese Vorrichtung 34 durch eine optische Lochblende gebildet. Die optische Lochblende weist gemäß der Erfindung neben einer kreisförmig begrenzten Öffnung für den Hauptstrahlengang des Beleuchtungslichts Öffnungen für den Off-Axis-Strahlengang des Beleuchtungslichts auf.

Die letztgenannten Öffnungen können eine kreisförmige Grundform haben, wobei es sich als vorteilhaft erwiesen hat, wenn diese von der kreisförmigen Grundform abweichend leicht asymmetrisch ausgebildet sind.

Im Strahlengang der Lichtquelle 18 ist in Strahlrichtung zwischen der Kollektor-Linsenanordnung 22 und der das pankratische System 26 aufweisenden Hilfslinsenanordnung eine Feldblende 36 angeordnet, die insbesondere als Irisblende ausgebildet sein kann. Die Kollektor-Linsenanordnung 22 bildet mit der Feldblende 36 und der Hilslinsenanordnung eine Köhler'sche Beleuchtung, bei der die Kollektor-Linsenanordnung 22 die Lichtquelle in die Ebene der Aperturblende 36 abbildet. Der Aufbau einer Köhler'schen Beleuchtung ist dem Fachmann allgemein bekannt und wird daher hier nicht näher erläutert.

Zur Vereinfachung der Darstellung sind in der Zeichnung die verschiedenen Optiken (Objektiv 6, Okular 16 Kollektor-Linsenanordnung 22) bzw. deren Teile (Variator 28, Kompensator 30) jeweils durch eine einzelne Linse in Form einer Sammellinse symbolisiert. Es versteht sich für den Fachmann von selbst, dass anstelle der symbolisch zu verstehenden einzelnen Sammellinse auch eine Mehrzahl von Linsen sowie eine Kombination und unterschiedlichen Linsen die jeweilige optische Baugruppe bilden kann.

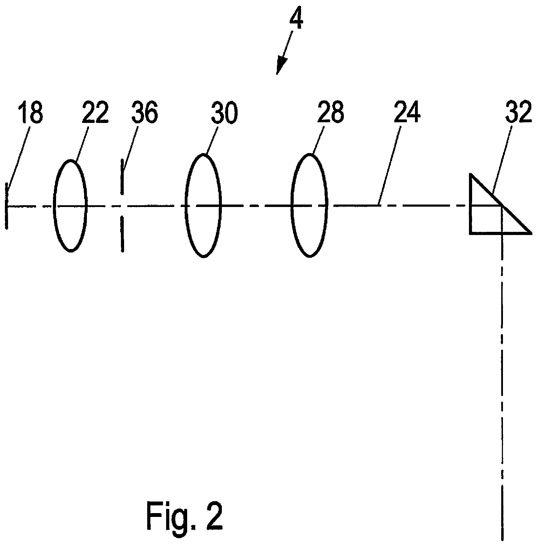

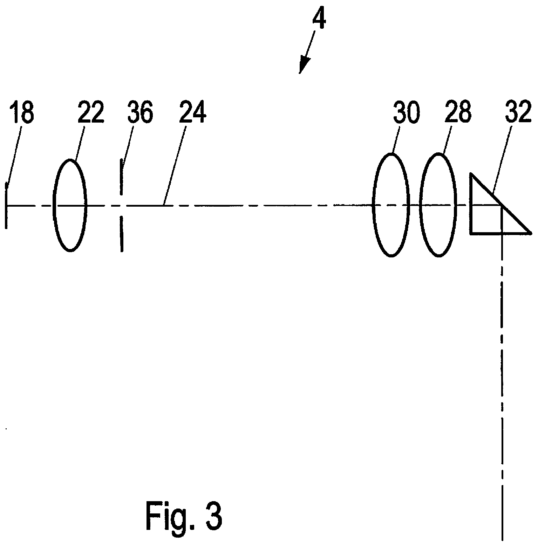

Die Funktionsweise der erfindungsgemäßen Beleuchtungsanordnung 4 wird nachfolgend unter Bezugnahme auf die Figuren 2 und 3 näher erläutert, die jeweils die Beleuchtungsanordnung 4 für sich genommen zeigen.

Fig. 2 zeigt das pankratische System 26 der Beleuchtungsoptik 20 in einer Brennweitenstellung, die der kürzesten Brennweite des pankratischen Systems 26 entspricht. In dieser Brennweitenstellung wird das Beobachtungsfeld des Mikroskops 2 entsprechend der Beleuchtungsstärke der Lichtquelle 18 homogen ausgeleuchtet.

Um die Beleuchtungsstärke im zentralen Bereich des Beobachtungsfeldes zu erhöhen, wird das pankratische System 26 zur Verlängerung von dessen Brennweite verstellt. Fig. 3 zeigt diejenige Brennweitenstellung des pankratischen Systems 26, die der längsten Brennweite entspricht. Hat das pankratische System 26 beispielsweise einen Vario-Faktor von 2, so ist die Brennweite in der in Fig. 3 dargestellten Brennweitenstellung doppelt so lang, wie in der in Fig. 2 dargestellten Brennweitenstellung. Da die Ausleuchtung in dem dann beleuchteten Teil des Beobachtungsfeldes weiterhin homogen ist, erhöht sich durch die Brennweitenverstellung die geometrische Beleuchtungsstärke um den Faktor 4.

Die erfindungsgemäße Beleuchtungsanordnung 4 ermöglicht es damit, bedarfsweise die Beleuchtungsstärke im Beobachtungsfeld wesentlich zu erhöhen. Somit können als Lichtquelle auch Leuchtdioden verwendet werden, deren Beleuchtungsstärke geringer ist, als es die im Sinne einer ausreichenden Beleuchtung maximal benötigte Beleuchtungsstärke im Beobachtungsfeld erfordern würde. Die Erfindung ermöglicht damit die Verwendung von relativ kostengünstigen Lichtquellen bei gleichzeitiger Aufrechterhaltung einer homogenen Ausleuchtung und Gewährleistung einer ausreichenden Beleuchtungsstärke im Beobachtungsfeld.

Geeignete Verstellmechaniken zur Betätigung der Zoom-Funktion des pankratischen Systems 26 sind dem Fachmann allgemein bekannt und werden daher hier nicht näher erläutert.

Bei aus dem Stand der Technik bekannten, nach Art einer Köhlerschen Beleuchtung ausgebildeten Beleuchtungsanordnungen ohne pankratisches System ist der Kollektor-Linsenanordnung 22 eine Hilfslinsenanordnung nachgeordnet. Ausgehend von einem derartigen Stand der Technik ist das erfindungsgemäß verwendete pankratische System 26 Teil der Hilfslinsenanordnung oder bildet die Hilfslinsenanordnung.

## Patentansprüche

1. Diagnose- oder Operationsmikroskop,
mit einer Beleuchtungsanordnung (4),
wobei die Beleuchtungsanordnung (4) eine Lichtquelle (18) aufweist und einen Beleuchtungsstrahlengang definiert, und
wobei die Beleuchtungsanordnung (4) eine zwischen der Lichtquelle (18) und einem einen Abbildungsstrahlengang definierenden Objektiv (6) des Mikroskops (2) angeordnete Beleuchtungsoptik (20) aufweist,
wobei im Strahlengang der Lichtquelle (18) wenigstens ein Umlenkmittel (32) zur Einkopplung des Beleuchtungsstrahlenganges in den Abbildungsstrahlengang angeordnet ist und
wobei die Beleuchtungsoptik (20) wenigstens ein pankratisches System (26) aufweist,
wobei die Beleuchtungsanordnung (4) in Strahlrichtung der Lichtquelle zwischen der Lichtquelle und dem Umlenkmittel angeordnet nach Art einer Köhler'schen Beleuchtung eine Kollektor-Linsenanordung (22), die in Strahlrichtung der Lichtquelle (18) hinter der Lichtquelle (18) angeordnet ist, eine der Kollektor-Linsenanordnung nachgeordnete Feldblende (36) und eine der Feldblende (36) nachgeordnete Hilfslinsenanordnung aufweist und
wobei die Hilfslinsenanordnung das wenigstens eine pankratische (26) System aufweist,
wobei
die Beleuchtungsanordnung (4) wenigstens eine optische Vorrichtung (34) zur Verringerung von Reflexionen des Beleuchtungslichts an dem Objektiv (6) des Mikroskops (2) aufweist,
**dadurch gekennzeichnet,**
**dass** die optische Vorrichtung (34) zur Verringerung von Reflexionen in Strahlrichtung der Lichtquelle (18) zwischen dem Umlenkmittel und dem Objektiv (6) des Mikroskops (2) angeordnet ist,
wobei optische Vorrichtung (34) zur Verringerung von Reflexionen wenigstens eine Lochblende aufweist und
wobei die Lochblende wenigstens eine kreisförmig begrenzte Öffnung für einen Beleuchtungs-Hauptstrahlengang der Beleuchtungsanordnung (4) sowie Öffnungen für einen Off-Axis-Strahlengang des Beleuchtungslichts aufweist.

2. Diagnose- oder Operationsmikroskop nach Anspruch 1, **dadurch gekennzeichnet, dass** die Lichtquelle (18) wenigstens eine Leuchtdiode aufweist.

3. Diagnose- oder Operationsmikroskop nach Anspruch 1 oder 2, **dadurch gekennzeichnet, dass** das Umlenkmittel (32) ein Prisma oder ein Spiegel ist.

4. Diagnose- oder Operationsmikroskop nach einem der vorhergehenden Ansprüche, **dadurch gekennzeichnet, dass** die Feldblende (36) als Irisblende ausgebildet ist.

## Claims

1. A diagnostic or surgical microscope,
with an illumination arrangement (4),
wherein the illumination arrangement (4) comprises a light source (18) and defines an illumination beam path, and
wherein the illumination arrangement (4) comprises an illumination optics (20) disposed between the light source (18) and an objective (6) of the microscope (2) defining an imaging beam path,
wherein at least one deflection means (32) for coupling the illumination beam path into the imaging beam path is disposed in the beam path of the light source (18), and
wherein the illumination optics (20) comprises at least one pancratic system (26),
wherein the illumination arrangement (4) comprises, in beam direction of the light source, between the light source and the deflection means, arranged in the manner of a Köhler illumination, a collector lens arrangement (22), which is disposed behind the light source (18) in beam direction of the light source (18), a field diaphragm (36) disposed downstream of the collector lens arrangement and an auxiliary lens arrangement disposed downstream of the field diaphragm (36), and
wherein the auxiliary lens arrangement comprises the at least one pancratic (26) system,
wherein the illumination arrangement (4) comprises at least one optical device (34) for reducing reflections of the illumination light at the objective (6) of the microscope (2),
**characterized in**
**that** the optical device (34) for reducing reflections in beam direction of the light source (18) is disposed between the deflection means and the objective (6) of the microscope (2),
wherein optical device (34) for reducing reflections comprises at least one pinhole diaphragm, and
wherein the pinhole diaphragm comprises at least one circularly limited opening for an illumination main beam path of the illumination arrangement (4) as well as openings for an off-axis beam path of the illumination light.

2. The diagnostic or surgical microscope according to claim 1, **characterised in that** the light source (18) comprises at least one illuminating diode.

3. The diagnostic or surgical microscope according to claim 1 or 2, **characterised in that** the deflection means (32) is a prism or a mirror.

4. The diagnostic or surgical microscope according to one of the preceding claims, **characterised in that** the field diaphragm (36) is formed as iris diaphragm.

## Revendications

1. Microscope de diagnostic ou d'opération,
avec un ensemble d'éclairage (4),
dans lequel l'ensemble d'éclairage (4) présente une source de lumière (18) et définit une trajectoire de faisceaux d'éclairage,
et
dans lequel l'ensemble d'éclairage (4) présente une optique d'éclairage (20) disposée entre la source de lumière (18) et un objectif (6), définissant une trajectoire de faisceaux de reproduction, du microscope (2),
dans lequel au moins un moyen de renvoi (32) destiné à injecter la trajectoire de faisceaux d'éclairage dans la trajectoire de faisceaux de reproduction est disposé sur la trajectoire de faisceaux de la source de lumière (18), et
dans lequel l'optique d'éclairage (20) présente au moins un système pancratique (26),
dans lequel l'ensemble d'éclairage (4), disposé dans la direction de rayonnement de la source de lumière, entre la source de lumière et le moyen de renvoi, à la manière d'un éclairage de Köhler, présente un ensemble lentille collecteur (22), qui est disposé dans la direction de rayonnement de la source de lumière (18) derrière la source de lumière (18), un diaphragme de champ (36) disposé en aval de l'ensemble de lentille collecteur, et un ensemble lentille auxiliaire disposé en aval du diaphragme de champ (36), et
dans lequel l'ensemble lentille auxiliaire présente l'au moins un système pancratique (26),
dans lequel
l'ensemble d'éclairage (4) présente au moins un dispositif optique (34) destiné à réduire des réflexions de la lumière d'éclairage sur l'objectif (6) du microscope (2),
**caractérisé en ce**
**que** le dispositif optique (34) destiné à réduire des réflexions est disposé dans la direction de rayonnement de la source de lumière (18) entre le moyen de renvoi et l'objectif (6) du microscope (2),
dans lequel le dispositif optique (34) destiné à réduire des réflexions présente au moins un diaphragme perforé, et
dans lequel le diaphragme perforé présente au moins une ouverture délimitée en forme de cercle pour une trajectoire de faisceaux principale d'éclairage de l'ensemble d'éclairage (4) ainsi que des ouvertures pour une trajectoire de faisceaux indirecte de la lumière d'éclairage.

2. Microscope de diagnostic et d'opération selon la revendication 1, **caractérisé en ce que** la source de lumière (18) présente au moins une diode électroluminescente.

3. Microscope de diagnostic et d'opération selon la revendication 1 ou 2, **caractérisé en ce que** le moyen de renvoi (32) est un prisme ou un miroir.

4. Microscope de diagnostic et d'opération selon l'une quelconque des revendications précédentes, **caractérisé en ce que** le diaphragme de champ (36) est réalisé en tant que diaphragme iris.
